# EUROPEAN PATENT SPECIFICATION

(11) **EP 1 678 500 B1**
(45) Date of publication and mention of the grant of the patent: **20.05.2009**
(21) Application number: 04791699.4
(22) Date of filing: 04.10.2004
(51) Int. Cl.: G01N 33/543, G01N 33/558, G01N 33/559

(54) **DIGITAL IMAGING OF SINGLE RADIAL IMMUNODIFFUSION ASSAYS**
ERSTELLUNG VON DIGITALAUFNAHMEN EINZELNER RADIALER IMMUNDIFFUSIONSTESTS
IMAGERIE NUMERIQUE DE DOSAGES SIMPLES PAR IMMUNODIFFUSION RADIAIRE

(30) Priority: 03.10.2003 GB 0323231
(43) Date of publication of application: 12.07.2006
(73) Proprietor: Novartis Vaccines and Diagnostics S.r.l., 53100 Siena (SI) (IT)
(72) Inventor: Di Pisa, Francesco, Chiron SRL, I-53100 Siena (IT)
(74) Representative: Marshall, Cameron John
(86) International application number: PCT/IB2004/003369
(87) International publication number: WO 2005/033695

(56) References cited:
- DE-A1- 4 040 726
- DE-A1- 4 318 692
- US-A1- 2001 053 368
- GUIDRY A J ET AL: "IMPROVED METHODOLOGY FOR QUANTITATIVE DETERMINATION OF SERUM AND MILK PROTEINS BY SINGLE RADIAL IMMUNO DIFFUSION" JOURNAL OF DAIRY SCIENCE, vol. 62, no. 8, 1979, pages 1252-1257, XP008043041 ISSN: 0022-0302

## Description

### TECHNICAL FIELD

This invention is in the field of radial immunodiffusion assays.

### BACKGROUND ART

The Single-Radial-Immuno-Diffusion (SRID) assay [refs. 1 & 2] for potency testing of influenza virus vaccines was originally developed by Wood *et al*. and was recommended by the WHO in 1978 to replace tests based on agglutination of erythrocytes. US2001/0053368A describes a proteosome influenze vaccine containing influenza HA.

The assay is based on diffusion of whole virus or purified viral antigens into agarose gel containing specific anti-hemagglutinin (anti-HA) serum. The resulting antigen/antibody reaction or zone is directly proportional to the amount of HA antigen in the preparation. By comparing the zones produced by an unknown preparation to the zones of a reference with known HA content (µg HA per ml), a value can be assigned to the unknown. Each reaction zone takes the form of concentric rings or circles. Each circle is characterized by its centre, diameter, and thickness (circular diffusion strip).

SRID assays have also been used to determine the potency of inactivated polio and rabies vaccines [1,2], using antibodies specific to the antigen being measured rather than anti-HA. More generally, the SRID assay can be used to determine the concentration of any antigen. For clinical testing purposes this concentration should be related to potency.

Historically, a semiautomatic apparatus [3] has been used to measure zone diameters in two orthogonal directions, with approximation to the nearest 0.1 mm. This method is slow, open to error and not fully accurate. Thus there remains a need for improved methods for performing SRID assays.

DE4318692A1 describes a method for examining antigen-containing blood serum samples by means of a single radial immune diffusion (SRID) by applying a mask over the surface of the gel which has holes corresponding to different concentrations. A hole in the mask is matched to a particular diffusion ring in the gel to provide a measurement of the concentration of the antibody which has formed the ring. DE4040726A describes a procedure for searching for a pattern formed by an immunological agglutination reaction on the bottom surface of a reaction vessel. Guidry (1979) Journal of Dairy Science 62:1252-1257 describes a quantitative determination of serum and milk proteins by a SRID.

### DISCLOSURE OF THE INVENTION

The invention provides a method for performing a SRID assay for an antigen or antibody of interest as defined by claim 1.

The method of the invention allows unambiguous optimal representation of immuno-complex rings, does not require human intervention, is not subject to operator influence, and allows images to be stored for future reference.

### The test sample, the antigen or antibody of interest, and the gel

The invention is used to determine the concentration of an antigen or antibody of interest.

Antigens are detected by including within the gel antibody specific for the antigen of interest. Conversely, antibody is detected by including within the gel the antibody recognised by the antibody of interest. Alternatively, an antibody may be detected as an antigen by using an anti-antibody antibody. In all cases, however, detection relies on the interaction between an antigen and an antibody specific for that antigen.

Antibody may be monoclonal or polyclonal. The use of antiserum containing polyclonal antibody is typical. When antibody is present within the gel, it will be present at a uniform concentration, which may or may not be known.

A preferred arrangement involves a gel containing antibody, which is used to determine the concentration of an antigen of interest within a test sample. A preferred antigen of interest is hemagglutinin (HA) from influenza virus, and thus a preferred gel is impregnated with anti-HA antibody. A preferred test sample is an influenza virus vaccine. Other test samples may comprise *H.influenzae* antigens, tetanus antigens, or other antigens found in pediatric vaccines.

The gel is preferably an agar or agarose gel, although other suitable materials are available and can be selected by the skilled person based on their ability to support radial diffusion of antigens or antibodies, and on their ability to support precipitation of antigen/antibody complexes.

### Radial diffusion

The gels used in SRID assays generally contain pre-defined wells into which test samples are loaded. The contents of the well then diffuse radially into the gel. As antigen/antibody diffuses outwards it meets a cognate antibody/antigen and initially forms a visible precipitate as a halo around the well. As diffusion continues, the concentration shifts towards excess substance and the precipitate dissolves. Diffusion continues further until the substance's concentration drops to allow precipitation again. Further diffusion allows precipitation, re-dissolving and re-precipitation until the substance is too low to re-dissolve the rim of the precipitate. The halo then stops increasing in diameter.

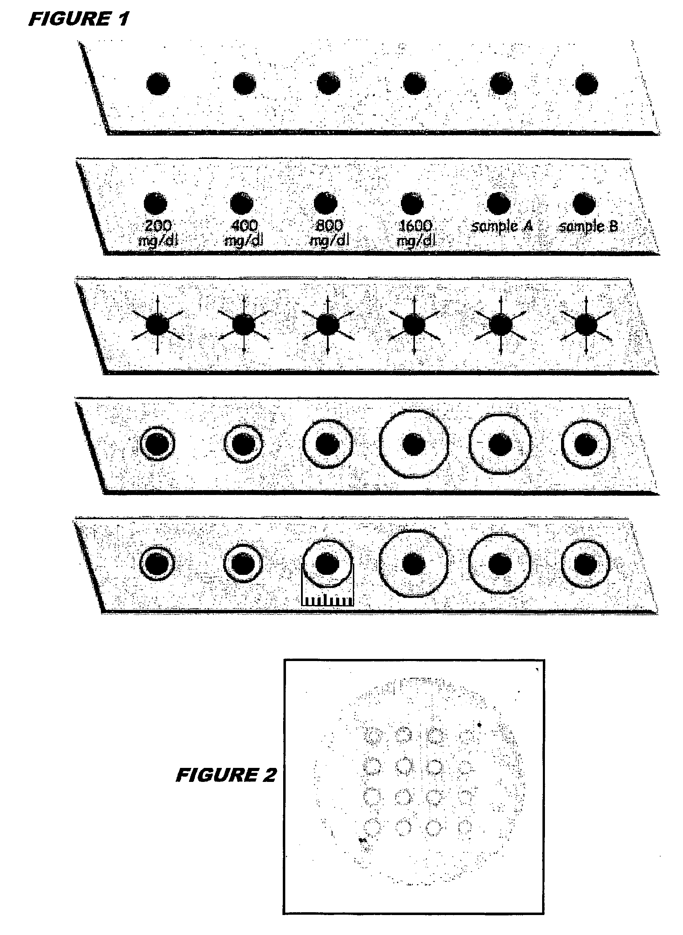

The result of the SRID assay (Figure 1) is thus a halo around the well, where the halo has an external and an internal diameter. The external diameter is proportional to the concentration of the test substance within the well. By comparing this diameter to the diameter of a halo obtained using a known concentration of substance, the SRID assay gives quantitative results.

### Digital imaging

After diffusion has taken place, step (c) of the method of the invention involves preparation of a digital image of the gel in a computer memory. The digital image need not be taken from the gel itself, but could be taken from a copy of the gel e.g. the gel could be photographed, and the image could be prepared from the resulting photographic paper.

The digital image can be obtained by scanning or by digital photography. The result will be a bitmap, which can be subjected to image processing techniques in step (d). The image may be a grey-scale image (to facilitate gradient analysis and edge detection) e.g. a 256-level grey-scale image. The image may have been subjected to image processing e.g. edge enhancement, *etc*.

Step (d) of the process involves analysing the digital image to locate features from the SRID assay results. These features may or may not be identified in a particular order. A first set of features to identify is the external diameter(s) of halo(s) formed by immunoprecipitation. These diameters are vital to determining concentration(s). A second set of features which may be identified is the internal diameter(s) of the halos, thereby defining an annulus. A third set of features which may be identified is the centre(s) of diffusion e.g. the location of wells in the gel.

It will be appreciated that a diameter can be measured directly or indirectly e.g. the diameter may be calculated after measuring a radius or an area.

It is rare that a SRID halo is a geometrically-perfect circle. An advantage of the invention is that an average diameter can be calculated for a halo as a whole rather than using a limited number of measurements. An overall area can be converted to the diameter of a circle with the same area. Locating internal diameters and/or diffusion centres assists such calculations. If diffusion occurs more on one side of a well than on the other, the inner open part of a halo may not be central within the external reaches of the halo (*i*.*e*. the inner and outer circles are not concentric), but locating the inner ring and/or centre of diffusion allow average diffusion in all radial directions to be assessed. Circular approximation functions may be used.

Locating halos within an image may be guided by features in the digital image. If the SRID assay uses a standard-sized gel with wells of known positioning, for example, and if the image contains standard recognition features, the positions of the wells can be inferred and image processing can work outwards from these positions to find halos.

Once halos have been identified within a digital image, further steps of the process may be performed on sub-images prepared from the overall image, each sub-image containing a small number of halos (typically a single halo per sub-image).

Image processing techniques for identifying and measuring ring or halo diameters are familiar to the skilled person. For example, image processing for iris recognition and characterisation is well known, and an immunoprecipitated halo is much less complex than an iris. To detect the edges of a halo against a background, the invention preferably utilises an algorithm for 2-D spatial gradient measurement within a greyscale image e.g. Sobel edge detection, Prewitt edge detection, Frei-Chen masks, *etc*.

### Diameter comparison and concentration determination

After the diameters of halos have been measured, they are compared to the diameters of halos formed using known concentrations of the substance in question. As the diameter of a halo is proportional to the concentration of a substance, this comparison allows the concentration of the substance in the test sample to be determined.

The method preferably involves a comparison with more than one such known diameter. A preferred SRID method obtains data using a variety of known concentrations. The diameters of halos formed from these concentrations are assessed, and then the two nearest standard halo diameters below and above are used to interpolate the concentration in a test sample. If the test halo is smaller or larger than the smallest/largest standard halos, such that two neighbours are not available, then at least the two closest diameters are used to extrapolate the concentration. As a zero concentration will give no halo, however, a zero value is always implicitly available for calculation purposes.

The method of the invention may thus involve the calculation of a standard curve, from which the concentration of a substance in a test sample may be interpolated or extrapolated.

These steps are generally performed using a computer.

The diameters of halos corresponding to the known concentrations are generally obtained from the same gel as the test halos *i*.*e.* the method of the invention will involve applying one or more standard samples containing known concentration(s) of an antigen/antibody of interest to the same gel as the test sample(s).

Within a single image it is important to know which halos arise from known concentrations and which arise from unknown concentrations. A user may have to select which halos correspond to specific standard concentrations. Alternatively, the method may use a specified arrangement of wells in a gel together with a specified arrangement of concentrations in specific wells.

Standard concentrations will be in the µg/ml range e.g. at least one standard concentration is between 1µg/ml and 500µg/ml.

### Image storage for future reference

The method of the invention may involve the further step of storing an image for future reference. This image is preferably stored together with information about the test sample e.g. a vaccine batch or lot number, a date, *etc*. Should a quality control issue arise with any particular batch, therefore, the SRID data can easily be retrieved. Data may be stored in a relational database.

Whole SRID images and/or sub-images (*e*.*g*. of individual halos) may be stored. If sub-images are stored it is preferred to cross-reference sub-images from the same gel.

Barcoding of samples and images can facilitate execution of the method and subsequence storage.

### Computer

Steps (c) to (f) of the method of the invention are generally performed by computer. Preferably, they are performed using the same computer, with the separate steps being performed by a single integrated software package.

Thus the invention provides a computer which program can analyse a digital image of a SRID gel after diffusion has taken place in order to locate the external diameter(s) of precipitation halos within the digital image and, optionally, locate (i) centre(s) of diffusion, and/or (ii) the internal diameter(s) of the halos, and which can compare the diameters to those obtained using one or more standard sample(s) containing a known concentration of a test substance, thereby calculating the concentration(s) of the substance which gave raise to the halos.

The invention also provides a storage medium (e.g. diskette, CD-ROM, memory, *etc*.) containing such a computer program.

The computer may allow visualisation of digital images, with and without superimposition of the position of located halos.

The invention also provides a system comprising a computer as described above and a scanner for obtaining digital images of SRID gels. The system may also comprise means for diluting and dispensing samples ready for SRID analysis. The system may provide visual indicators to a user to instruct them which samples to place in which wells during preparation of the SRID gel.

The invention also provides a computer programmed to perform steps (c) to (f) of the method.

### General

The term "comprising" means "including" as well as "consisting" *e*.*g*. a composition "comprising" X may consist exclusively of X or may include something additional *e*.*g*. X + Y.

The term "about" in relation to a numerical value x means, for example, x±10%.

### BRIEF DESCRTPTION OF THE DRAWINGS

Figure 1 shows the principles of the SRID assay.
Figures 2-4 show a SRID analysis from gel to processed image.
Figure 5 shows a processed image of a SRID gel.
Figures 6-8 show a SRID analysis from gel to processed image.
Figure 9 shows a processed SRID gel image, and Figure 10 shows the corresponding standard curve.

### MODES FOR CARRYING OUT THE INVENTION

### Example 1 - influenza virus

A standard SRID assay for influenza hemagglutinin was performed using a 4x4 matrix of wells in a plate. Standards were loaded in the top row at normal concentration, 2/3 dilution, 1/2 dilution and 1/4 dilution (left-to-right). Unknowns were loaded in rows 2 to 4 at the same dilutions.

The assay results were analysed using a Java-based software package which integrated scanning, image manipulation and Oracle^{™} database entry.

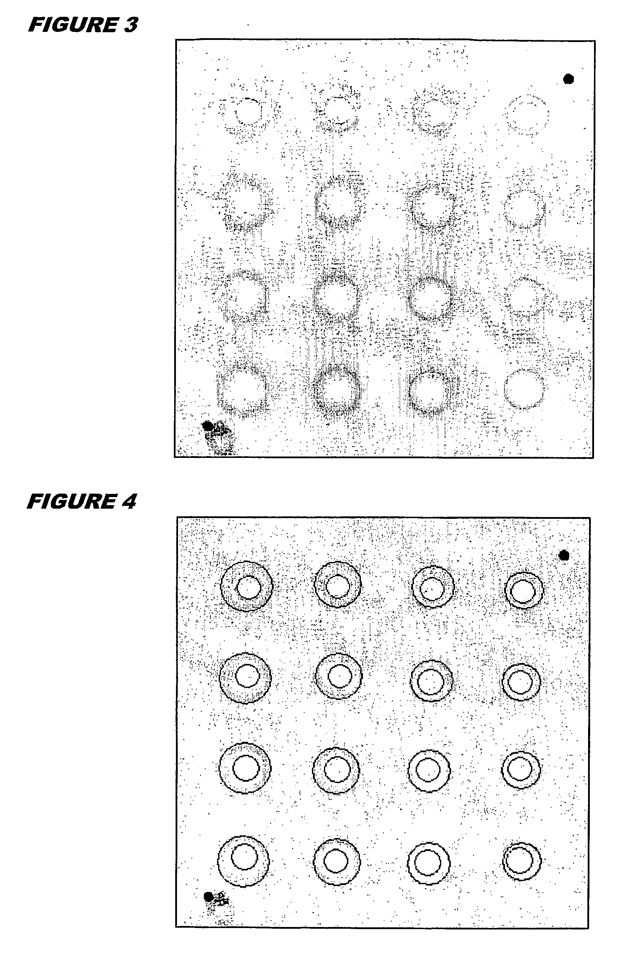

A digital image of the plate was captured using a scanner, as shown in Figure 2. The region of interest in the gel was automatically selected by software (Figure 3). The halos in this region were detected by software using image processing algorithms, and the calculated inner and outer diameters were superimposed on the visual representation shown to a user (Figure 4).

Outer circular diameters were measured in arbitrary units in two orthogonal directions, and results are shown below:

| **Row** | **Column** | **Diameter (1)** | **Diameter (2)** | **Product (1)x(2)** |
|---|---|---|---|---|
| 1 | 1 | 5.33 | 5.33 | 28.40 |
| | 2 | 4.69 | 4.69 | 21.99 |
| | 3 | 4.42 | 4.42 | 19.56 |
| | 4 | 3.84 | 3.84 | 14.72 |
| 2 | 1 | 4.69 | 4.69 | 21.99 |
| | 2 | 4.42 | 4.42 | 19.56 |
| | 3 | 4.26 | 4.26 | 18.18 |
| | 4 | 3.84 | 3.84 | 14.72 |
| 3 | 1 | 4.90 | 4.90 | 24.04 |
| | 2 | 4.48 | 4.48 | 20.04 |
| | 3 | 4.05 | 4.05 | 16.40 |
| | 4 | 3.78 | 3.78 | 14.32 |
| 4 | 1 | 4.85 | 4.85 | 23.52 |
| | 2 | 4.42 | 4.42 | 19.56 |
| | 3 | 4.21 | 4.21 | 17.72 |
| | 4 | 3.78 | 3.78 | 14.32 |

A standard curve was calculated from the values in row 1. Its slope is 18.64 and its intercept is 15.90, with a r²=0.9988. Using this standard curve, the HA concentrations of the samples used for rows 2 to 4 were calculated: (2) 16.78µg/ml; (3) 23.34µg/ml; (4) 21.38µg/ml.

### Example 2 - alternative influenza virus analysis

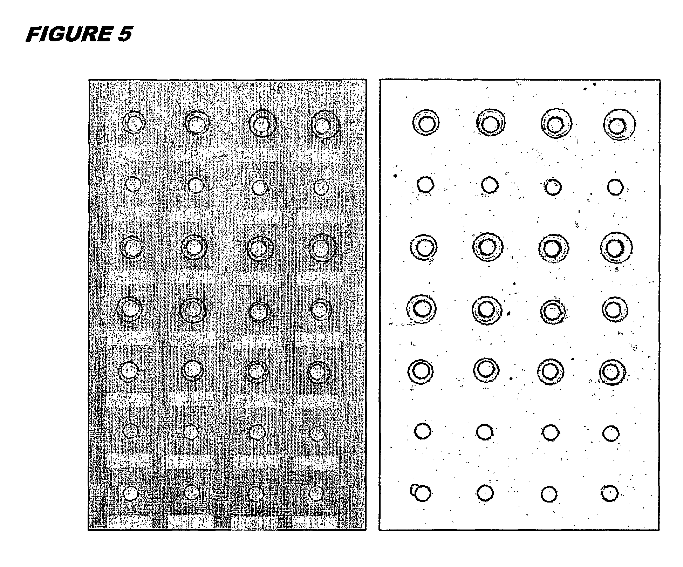

Rather than use a 4x4 format, a 4x7 format was used (Figure 5). Image filtering was also used to remove background (compare left and right), and some manual tuning was possible.

### Example 3 - H.influenzae type b antigens

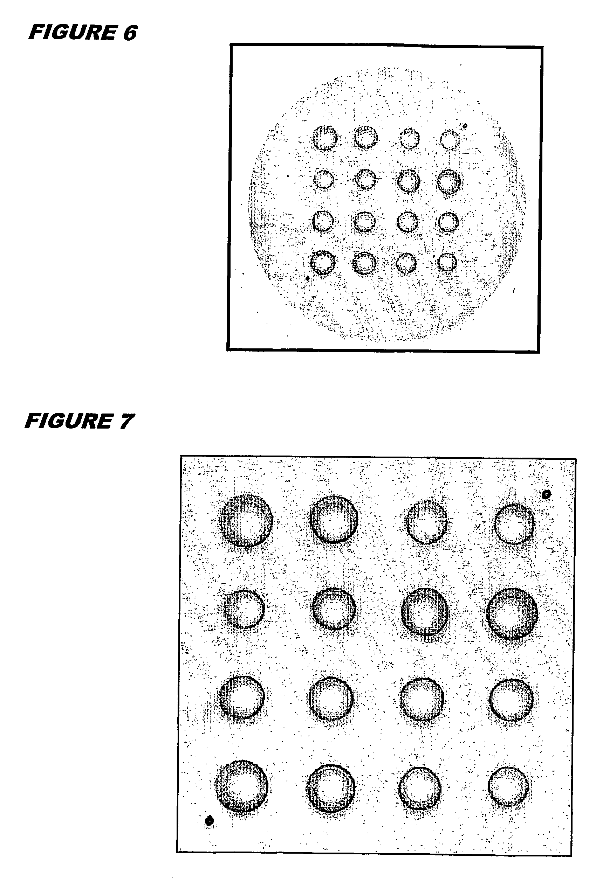

Figures 6 and 7 show the raw SRID assay and the auto-selected region of interest using conjugated *H.influenzae* type b (Hib) antigen in a anti-Hib gel.

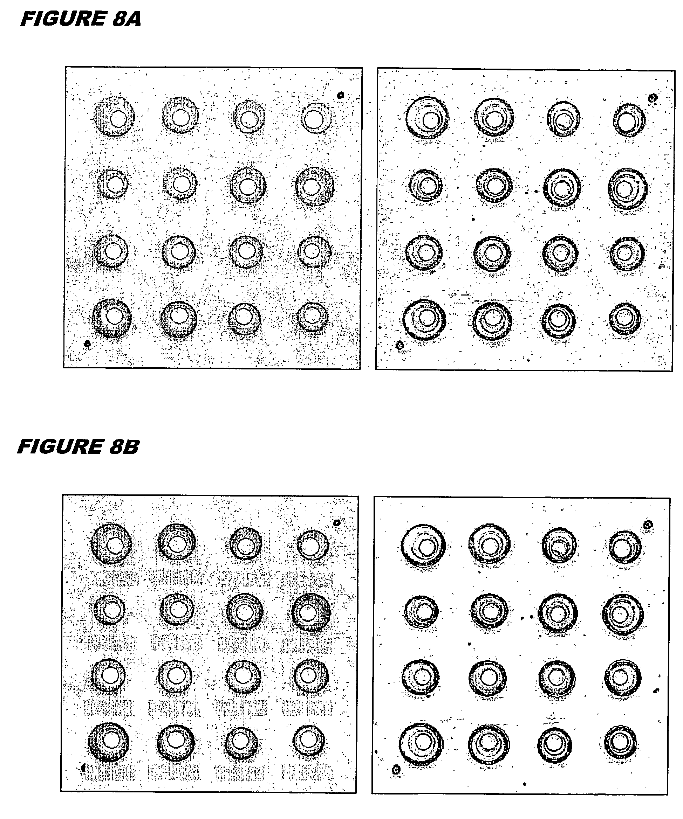

Figure 8A shows the use of image filtering to enhance edges, automatic inner-ring detection, inner-ring size determination, plus intermediate-ring location. In contrast, Figure 8B shows automatic outer-ring detection and size determination.

### Example 4 - tetanus toxoid

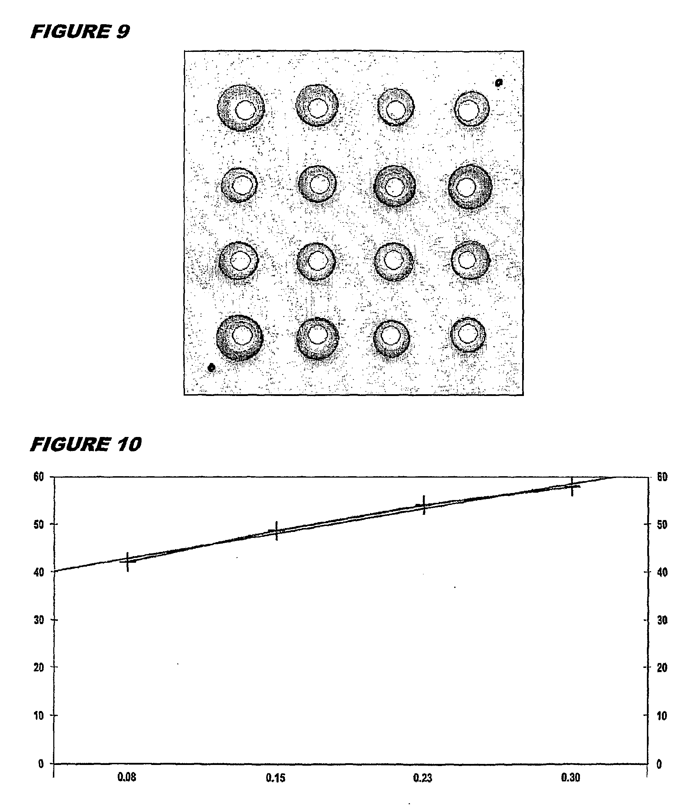

The final result of a tetanus toxoid (TT) SRID analysis using intermediate rings is shown in Figure 9, and graphical analysis of the results is shown in Figure 10 ( y = 70.044 x + 37.517 , r² = 0.9866 ). A test sample was calculated to contain 12.4 Lf/ml.

### REFERENCES (the contents of which are hereby incorporated in full)

[1] Williams (1993) Vet Microbiol 37:253-262.
[2] Fitzgerald & Needy (1986) Dev Biol Stand 64:73-79.
[3] US patent 4,099,881.

## Claims

1. A method for performing a SRID assay for an antigen or antibody of interest, comprising the steps of:
(a) applying one or more test sample(s) and one or more standard sample(s) to a gel, wherein the gel contains (i) antibody specific to the antigen of interest and/or (ii) antigen recognised by the antibody of interest, wherein the standard sample(s) contain(s) a known concentration of the antigen or antibody of interest;
(b) allowing the test and standard samples to diffuse radially from their points of application;
(c) preparing a digital image of the gel after diffusion has taken place;
(d) determining the external diameter(s) of precipitation halos within the digital image by determining the overall area of the precipitation halos and calculating therefrom diameter(s) of circle(s) with the same area;
(e) comparing the determined diameters resulting from the test sample(s) to those resulting from the standard sample(s); and, optionally,
(f) using the results from step (e) to determine the concentration(s) of the antigen or antibody of interest in the test sample(s).

2. The method of claim 1, wherein the assay is for an antigen of interest, and wherein the gel contains antibody.

3. The method of claim 2, wherein the antigen of interest is hemagglutinin from influenza virus.

4. The method of claim 3, wherein the gel is impregnated with anti-hemagglutinin antibody.

5. The method of claim 3 or claim 4, wherein the test sample is an influenza virus vaccine.

6. The method of any preceding claim, wherein the gel is an agar or agarose gel.

7. The method of any preceding claim, wherein the gel contains wells.

8. The method of any preceding claim, wherein the digital image is obtained by scanning or by digital photography.

9. The method of any preceding claim, wherein the image is a grey-scale image.

10. The method of any preceding claim, wherein the digital image is subjected to a 2-D spatial gradient measurement.

11. The method of claim 10, wherein the gradient measurement is Sobel edge detection, Prewitt edge detection, or a Frei-Chen mask.

12. The method of any preceding claim, wherein the gel contains antigen or antibody at a variety of known concentrations, in addition to the test sample.

13. The method of any preceding claim, comprising the further step of storing an image for future reference.

14. A computer programmed to analyse a digital image of a SRID gel after diffusion has taken place in order to locate the external diameter(s) of precipitation halos within the digital image and locate (i) centre(s) of diffusion, and/or (ii) the internal diameter(s) of the halos, and which determines the external diameter(s) of the halos by determining the overall area of the precipitation halos and calculating therefrom diameter(s) of circle(s) with the same area; compares the determined diameters to those obtained using one or more standard sample(s) applied to the gel containing a known concentration of a test substance, thereby calculating the concentration(s) of the substance which gave raise to the halos.

15. A system comprising the computer of claim 14, and a scanner for obtaining digital images of SRID gels.

## Patentansprüche

1. Verfahren zur Durchführung eines SRID-Assays, der auf ein Antigen oder einen Antikörper von Interesse gerichtet ist und Schritte umfasst, bei denen man:
(a) eine oder mehrere Testprobe(n) und eine oder mehrere Standard-Probe(n) auf ein Gel aufträgt, wobei das Gel (i) einen Antikörper umfasst, der spezifisch für das Antigen von Interesse ist, und/oder (ii) ein Antigen, das von dem Antikörper von Interesse erkannt wird, wobei die Standard-Probe(n) eine bekannte Konzentration des Antigens oder des Antikörpers von Interesse enthält (enthalten);
(b) es den Test- und den Standard-Proben erlaubt, radial von ihren Auftragungspunkten zu diffundieren;
(c) ein digitales Bild des Gels herstellt, nachdem die Diffusion stattgefunden hat;
(d) den (die) äußeren Durchmesser der Präzipitationshöfe innerhalb des digitalen Bildes bestimmt, indem man die Gesamtfläche der Präzipitationshöfe bestimmt und daraus den (die) Durchmesser des Kreises (der Kreise) mit der gleichen Fläche berechnet;
(e) die bestimmten Durchmesser, die sich aus der (den) Testprobe(n) ergeben mit denjenigen vergleicht, die anhand der Standard-Probe(n) erhalten wird (werden); und wahlweise
(f) die Ergebnisse von Schritt (e) verwendet, um die Konzentration(en) des Antigens oder des Antikörpers von Interesse in der (den) Testprobe(n) zu bestimmen.

2. Verfahren nach Anspruch 1, wobei der Assay auf ein Antigen von Interesse gerichtet ist, und wobei das Gel einen Antikörper enthält.

3. Verfahren nach Anspruch 2, wobei das Antigen von Interesse Hämagglutinin von Influenza-Virus ist.

4. Verfahren nach Anspruch 3, wobei das Gel mit Anti-Hämagglutinin-Antikörper imprägniert ist.

5. Verfahren nach Anspruch 3 oder Anspruch 4, wobei die Testprobe ein Influenza-Virus-Impfstoff ist.

6. Verfahren nach einem der vorhergehenden Ansprüche, wobei das Gel ein Agar oder ein Agarose-Gel ist.

7. Verfahren nach einem der vorhergehenden Ansprüche, wobei das Gel Vertiefungen enthält.

8. Verfahren nach einem der vorhergehenden Ansprüche, wobei das digitale Bild durch Scannen oder durch Digitalphotographie erhalten wird.

9. Verfahren nach einem der vorhergehenden Ansprüche, wobei das Bild ein Graustufenbild ist.

10. Verfahren nach einem der vorhergehenden Ansprüche, wobei das digitale Bild einer räumlichen 2D-Gradientenmessung unterzogen wird.

11. Verfahren nach Anspruch 10, wobei die Gradientenmessung eine Sobel-Kantenerkennung, eine Prewitt-Kantenerkennung oder eine Frei-Chen-Maske ist.

12. Verfahren nach einem der vorhergehenden Ansprüche, wobei das Gel neben der Testprobe Antigen oder Antikörper in einer Vielzahl von bekannten Konzentrationen umfasst.

13. Verfahren nach einem der vorhergehenden Ansprüche, das ferner den Schritt der Speicherung eines Bildes als künftige Referenz umfasst.

14. Computer, der dazu programmiert ist, ein Digitalbild eines SRID-Gels nach stattgefundener Diffusion zu analysieren, um den (die) äußeren Durchmesser der Präzipitationshöfe innerhalb des digitalen Bildes zu lokalisieren, und um (i) das Zentrum (die Zentren) der Diffusion und/oder (ii) den (die) äußeren Durchmesser der Höfe zu lokalisieren, und der den (die) äußeren Durchmesser der Höfe bestimmt, indem er die Gesamtfläche der Präzipitationshöfe bestimmt und daraus den (die) Durchmesser des Kreises (der Kreise) mit der gleichen Fläche berechnet; wobei dieser die bestimmten Durchmesser mit denjenigen vergleicht, die unter Verwendung von einer oder mehreren auf das Gel aufgetragenen Standard-Proben erhalten werden, und die eine bekannte Konzentration der Testsubstanz enthalten, wodurch die Konzentration(en) der Substanz berechnet wird (werden), die zu den Höfen geführt hat (haben).

15. System, das den Computer nach Anspruch 14 sowie einen Scanner zum Erhalt von digitalen Bildern von SRID-Gelen umfasst.

## Revendications

1. Méthode destinée à exécuter un dosage SRID (Immunodiffusion radiale simple) pour un antigène ou un anticorps d'intérêt, comprenant les étapes consistant à :
(a) appliquer un ou plusieurs échantillon(s) test et un ou plusieurs échantillon(s) standard sur un gel, dans laquelle le gel contient (i) un anticorps spécifique à l'antigène d'intérêt et/ou (ii) un antigène reconnu par l'anticorps d'intérêt, dans laquelle le(s) échantillon(s) standard contient (contiennent) une concentration connue de l'antigène ou de l'anticorps d'intérêt ;
(b) permettre aux échantillons test et standard de diffuser radialement à partir de leurs points d'application ;
(c) préparer une image numérique du gel après la diffusion ;
(d) déterminer le(s) diamètre(s) externe(s) des halos de précipitation sur l'image numérique en déterminant l'aire totale des halos de précipitation et en calculant à partir de ce résultat le (s) diamètre (s) du (des) cercles de même aire ;
(e) comparer les diamètres déterminés à partir de(s) (l')échantillon(s) test à ceux provenant de(s) (l')échantillon(s) standard ; et, éventuellement,
(f) utiliser les résultats obtenus à l'étape (e) pour déterminer le(s) concentration(s) de l'antigène ou de l'anticorps d'intérêt dans l'(les) échantillon(s) test.

2. Méthode selon la revendication 1, dans laquelle le dosage concerne un antigène d'intérêt, et dans laquelle le gel contient un anticorps.

3. Méthode selon la revendication 2, dans laquelle l'antigène d'intérêt est une hémagglutinine provenant du virus de la grippe.

4. Méthode selon la revendication 3, dans laquelle le gel est imprégné d'un anticorps anti-hémagglutinine.

5. Méthode selon la revendication 3 ou 4, dans laquelle l'échantillon test est un vaccin antigrippal.

6. Méthode selon l'une quelconque des revendications précédentes, dans laquelle le gel est un gel d'agar ou d'agarose.

7. Méthode selon l'une quelconque des revendications précédentes, dans laquelle le gel contient des puits.

8. Méthode selon l'une quelconque des revendications précédentes, dans laquelle l'image numérique s'obtient par scanner ou par photographie numérique.

9. Méthode selon l'une quelconque des revendications précédentes, dans laquelle l'image est une image d'échelle de gris.

10. Méthode selon l'une quelconque des revendications précédentes, dans laquelle l'image numérique est soumise à une mesure du gradient spatial en 2D.

11. Méthode selon la revendication 10, dans laquelle la mesure du gradient se fait soit par détection des contours de Sobel, soit par détection des contours de Prewitt, ou en utilisant le masque de Frei-Chen.

12. Méthode selon l'une quelconque des revendications précédentes, dans laquelle le gel contient des antigènes ou des anticorps dont on connaît les différentes concentrations, en plus de l'échantillon test.

13. Méthode selon l'une quelconque des revendications précédentes, comprenant l'étape supplémentaire consistant à stocker une image afin de pouvoir s'y référer à l'avenir.

14. Ordinateur programmé pour l'analyse d'une image numérique ou d'un gel SRID après que la diffusion ait eu lieu afin de localiser le(s) diamètre(s) externe(s) des halos de précipitation sur l'image numérique et (i) le(s) centre(s) de diffusion, et/ou (ii) le(s) diamètre(s) internes des halos, et qui détermine le(s) diamètre(s) externe(s) des halos en déterminant l'aire totale des halos de précipitation et en calculant à partir de ce résultat le(s) diamètre(s) du(des) cercle (s) de même aire ; comparer les diamètres déterminés à ceux obtenus en utilisant un ou plusieurs échantillon(s) standard appliqué(s) au gel contenant une concentration connue d'une substance test, en calculant le(s) concentration(s) de la substance à l'origine de la formation des halos .

15. Système comprenant l'ordinateur selon la revendication 14, et un scanner permettant d'obtenir des images numériques des gels SRID.
